# EUROPÄISCHE PATENTANMELDUNG

(11) **EP 1 050 524 A2**
(43) Veröffentlichungstag der Anmeldung: **08.11.2000**
(21) Anmeldenummer: 00109004.2
(22) Anmeldetag: 26.04.2000
(51) Int. Cl.: C07C 43/11, C07C 41/16, C08G 65/32, C11D 1/72

(54) **Verfahren zur Herstellung von alkyl-endgruppenverschlossenen Alkyl- und/oder Alkenylpolyglycolethern**

(30) Priorität: 05.05.1999 DE 19920559
(71) Anmelder: Cognis Deutschland GmbH, 40589 Düsseldorf (DE)
(72) Erfinder: Tundo, Pietro, Prof., 30172 Chirignago Venezia (IT); Memoli, Sofia, Dr., 30030 Chirignago Venezia (IT); Herault, David, Dr., 50670 Köln (DE); Löhl, Thorsten, Dr., 53179 Bonn (DE)

(57) **Zusammenfassung**

Vorgeschlagen wird ein Verfahren zur Herstellung von alkyl-endgruppenverschlossenen Alkyl- und/oder Alkenylethern, **dadurch gekennzeichnet**, daß man Alkohole der Formel **(I)**, 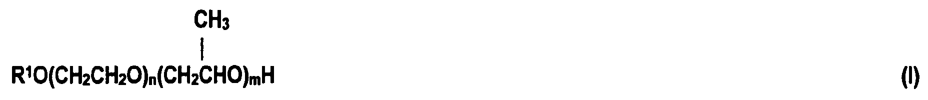 in der R¹ für einen linearen oder verzweigten, gesättigten oder ungesättigten Alkyl-, Alkenyl- und/oder Acylrest mit 6 bis 22 Kohlenstoffatomen und/oder gegebenenfalls mit einer Gruppe R² substituierten Kohlenwasserstoffrest, in der R² für H, C₁₋₁₂ Alkyl, Glyceryl oder den Rest eines Alkylglycols mit 2 bis 6 Kohlenstoffatomen, n für Zahlen von 0 bis 15 und m für Zahlen von 0 bis 10 steht, mit Dialkylcarbonat der Formel **(II)**, 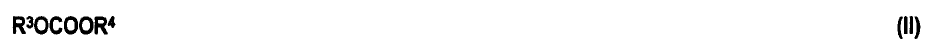 in der R³ und R⁴ unabhängig voneinander für einen Alkylrest mit 1 bis 8 Kohlenstoffatomen steht, umsetzt.

## Beschreibung

### Gebiet der Erfindung

Die Erfindung betrifft ein Verfahren zur Herstellung von alkyl-endgruppenverschlossenen Alkyl- und/ oder Alkenylethern und deren Verwendung zur Herstellung von schaumarmen Reinigungsmitteln sowie Entschäumern für die Zucker- und Hefeindustrie.

### Stand der Technik

Für eine Vielzahl technischer Prozesse ist die Anwesenheit von Schaum äußerst unerwünscht. So kommt es z. B. sowohl bei der maschinellen Reinigung von Bier- oder Milchflaschen als auch der Spritzreinigung von Automobilblechen nicht nur auf die reinigende bzw. entfettende Wirkung der eingesetzten oberflächenaktiven Mittel an, die Vermeidung von Schaum, der die Anlagenfunktionen stark beeinträchtigen kann, ist von gleich großer Bedeutung. Dies gilt um so mehr, als in vielen Fällen hochaktive, jedoch auch starkschäumende anionische Tenside eingesetzt werden.

Das Problem der Schaumregulierung ist freilich seit langer Zeit bekannt und dementsprechend sind aus dem Stand der Technik eine Vielzahl von mehr oder weniger überzeugenden Problemlösungen bekannt, die in zwei Gruppen eingeteilt werden können:

Bei der einen handelt es sich um Verfahren unter Zugabe von Entschäumern, bei denen es sich vielfach um paraffinische Kohlenwasserstoffe oder Siliconverbindungen handelt. Für die beschriebenen Anwendungen ist dies jedoch in den meisten Fällen unerwünscht. Bei der zweiten Gruppe von Verfahren handelt es sich um den Einsatz von oberflächenaktiven Mitteln, die selbst schaumarm sind und gegebenenfalls zusätzlich noch über entschäumende Eigenschaften verfügen. In der Regel handelt es sich hierbei um nichtionische Tenside oder tensidähnliche Systeme, wie beispielsweise Fettalkoholpropylenglycolether oder Blockpolymere von Ethylen- und Propylenglycol, die allerdings keine ausreichende biologische Abbaubarkeit aufweisen.

Als besonders effektive schaumarme Tenside haben sich endgruppenverschlossene Fettalkoholpolyglycolether, sogenannte 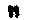 Mischether" am Markt etabliert, die beispielsweise von R. Piorr in **Fat.Sci. Technol. 89, 106 (1987)** beschrieben werden. Bei diesen Produkten handelt es sich in der Regel um butylendgruppenverschlossene Niotenside, wie sie beispielsweise aus den Schriften **EP 0124815 A**, **EP 0303928 B, EP 0324340 B, EP 0420802 A, DE 3928600 A** und **DE 4243643 C** bekannt sind.

Eine Sonderstellung nehmen Methylmischether ein, die einen Methyl-Endgruppenverschluß aufweisen und üblicherweise durch Umsetzung der entsprechenden Fettalkoholpolyglycolether mit Methylhalogeniden [**US 4,587,365**, BASF] oder Dimethylsulfat [**EP 0302487 B**, BASF] hergestellt werden.

In der **EP 0476784** (Union Carbide) ist die Herstellung von methyl-endgruppenverschlossenen Alkylethern unter Verwendung von Dimethylcarbonat beschrieben. Dimethylcarbonat ist ein mildes und umweltfreundliches Alkylierungsmittel im Vergleich zu Dimethylsulfat oder Alkylhalogeniden [M. Lissel, A. R. Rohani-Dezfuli in Kontakte (Darmstadt) 1990 (1), 20-23]. Dieses Verfahren zeichnet sich jedoch durch niedrigem Ausbeute und ein breites Produktspektrum aus. Ein weitgehend vollständiger Verschluß ist notwendig um die Anwendbarkeit des Produktes als schaumarmes Tensid zu gewährleisten.

So ist beispielsweise aus der **EP 0161537 A** (BASF) die Verwendung u.a. von methyl-endgruppenverschlossenen Mischethem für die maschinelle Flaschenreinigung bekannt. In der **EP 0180081 B** (BASF) werden die gleichen Stoffe als Entschäumer für die Zucker- und Hefeindustrie vorgeschlagen. Gegenstand der **EP 0202638 B** (BASF) sind schließlich flüssige Tensidkonzentrate für stark alkalische Reinigungsmittel, enthaltend Methylmischether, Alkylpolyglucoside, Anlagerungsprodukte von Maleinsäureanhydrid an ungesättigte Fettsäuren sowie verzweigte Fettsäuren.

Gegenstand der **US 4,624,803** (Hüls) ist der Einsatz von endgruppenverschlossenen Fettalkoholalkoxylaten als schaumarme Reinigungsmittel für die maschinelle Flaschenreinigung. In Beispiel 10 wird ein methyl-endgruppenverschlossenes Anlagerungsprodukt von 9 Mol Ethylenoxid und 2 Mol Propylenoxid an einen Oxoalkohol mit 13 bis 15 Kohlenstoffatomen beschrieben, welches jedoch nur unzureichende Entschäumereigenschaften aufweist.

Aus der **DE 4431158 A1** (Henkel) sind methyl-endgruppenverschlossene Alkylpolyglycolether bekannt, bei deren Herstellung zusätzlich zu den Methylierungsmitteln Basen eingesetzt werden müssen.

Die Alkylether können aus den bekannten Verfahren entweder in unzureichenden Ausbeuten oder unter Einsatz von starken Alkylierungsmittel und durch Zugabe von Basen hergestellt werden. Diese starken Alkylierungsmittel sind aufgrund der notwendigen besonderen Vorsichtsmaßnahmen bei den Produktion von Nachteil, da diese eine gesundheitliche und ökologische Gefahr darstellen. Darüber hinaus haben Alkylmischether bezüglich des Schaumvermögens und insbesondere Entschäumungsverhaltens gegenüber den weit verbreiteten Butylmischethern stets deullich schlechter abgeschnitten. Infolge der limitierten Verfügbarkeit von Butylchlorid als geeignetem Reagenz für den C₄-Endgruppenverschluß von Alkylpolyglycolethern hat das Marktbedürfnis nach Alkylmischethern mit verbesserten anwendungstechnischen Eigenschaften, die einfach, salzfrei, preiswert und mit hohen Ausbeuten herzustellen sind, in den letzten Jahren jedoch stark zugenommen.

Die Aufgabe der Erfindung hat somit darin bestanden, alkyl-endgruppenverschlossenen Alkylethern einfach, salzfrei, preiswert und mit hohen Ausbeuten herzustellen. Darüber hinaus sollen die so hergestellten Alkylpolyglycolether frei von den geschilderten Nachteilen sein und sich durch ein geringes eigenes Schaumvermögen, gute Reinigungsleistung und zufriedenstellende biologische Abbaubarkeit auszeichnen.

### Beschreibung der Erfindung

Gegenstand der Erfindung ist ein Verfahren zur Herstellung von alkyl-endgruppenverschlossenen Alkyl- und/oder und/oder Alkenylethern, **dadurch gekennzeichnet**, daß man Alkohole der Formel **(I)**, 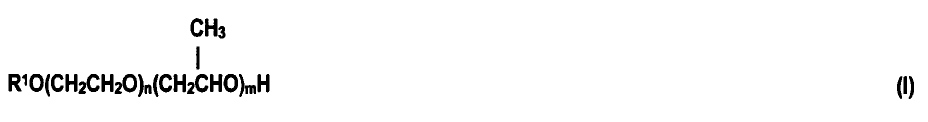 in der R¹ für einen linearen oder verzweigten, gesättigten oder ungesättigten Alkyl-, Alkenyl- und/oder Acylrest mit 6 bis 22 Kohlenstoffatomen und/oder gegebenenfalls mit einer Gruppe R² substituierten Kohlenwasserstoffrest, in der R² für H, C₁₋₁₂ Alkyl, Glyceryl oder den Rest eines Alkylglycols mit 2 bis 6 Kohlenstoffatomen, n für Zahlen von 0 bis 15 und m für Zahlen von 0 bis 10 steht, mit Dialkylcarbonat der Formel **(II)**, 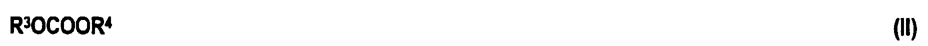 in der R³ und R⁴ unabhängig voneinander für einen Alkylrest mit 1 bis 8 Kohlenstoffatomen steht, umsetzt.

Überraschenderweise wurde gefunden, daß sich alkyl-endgruppenverschlossene Alkylether einfach, salzfrei, preiswert und mit hohen Ausbeuten unter Verwendung eines milden Alkylierungsmittels herzustellen lassen. Die so erhaltenen Alkylmischether weisen gute schaumdrückende Eigenschaften auf und zeichnen sich ferner durch ein sehr geringes eigenes Schaumvermögen, gute Reinigungsleistung und zufriedenstellende biologische Abbaubarkeit aus.

### Alkoholethoxylate

Es können Alkohole der Formel **(I)** eingesetzt werden, in der R¹ für einen Alkyl- und/oder Alkenylrest mit 6 bis 22 und vorzugsweise 8 bis 18 Kohlenstoffatomen, n für Zahlen von 0 bis 15, vorzugsweise 1 bis 14, und insbesondere 4 bis 5 und m für Zahlen von 0 bis 10, vorzugsweise 1 bis 9, und insbesondere 2 bis 5 steht. Fettalkoholethoxylate werden bekanntlich durch Anlagerung von n Mol Ethylenoxid und anschließend m Mol Propylenoxid an Fettalkohole erhalten.
Typische Beispiele für Fettalkohole sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalko- hol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Alkylestern auf Basis von Fellen und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol. Als Fettalkoholethoxylate kommen ferner **Guerbetalkoholethoxylate** der Formel **(I)** in Frage, in der R¹ für einen in 2-Stellung verzweigten Alkylrest mit 16 bis 20 Kohlenstoffatomen steht. Guerbetalkohole werden üblicherweise durch basenkatalysierte Kondensation von Fettalkoholen mit anschließender Anlagerung von n Mol Ethylenoxid und m Mol Propylenoxid an Fettalkohole hergestellt. Eine Übersicht zu dem Thema ist von A. J. O'Lennick und R. E. Bilbo in **Soap Cosm.Chem.Spec. April, 52 (1987)** erschienen. Typische Beispiele sind Anlagerungsprodukte von 2-Hexyldodecanol und 2-Octyltetradecanol sowie deren Mischungen auf Basis von technischen C₈-C₁₀-Fettalkoholen. Die letztgenannten Produkte zeichnen sich durch ein besonders hohes Reinigungsvermögen und sehr gute biologische Abbaubarkeit aus.

### Ethoxylierung und Propoxylierung

Die Ethoxylierung und Propoxylierung der genannten Alkohole kann in an sich bekannter Weise bei Temperaturen von 120 bis 180 °C in Anwesenheit basischer Katalysatoren wie beispielsweise Natriumalkylat vorzugsweise Natriummethylat oder calciniertem Hydrotalcit durchgeführt werden. Demzufolge können die Alkoxylate sowohl eine konventionelle als auch eine eingeengte Homologenverteilung aufweisen. Es sei noch einmal ausdrücklich darauf hingewiesen, daß die Abfolge von Ethoxylierung und Propoxylierung kritisch für die anwendungstechnischen Eigenschaften der Produkte ist. Alkyl- vorzugsweise methyl-endgruppenverschlossene Mischether, die im Sinne der Erfindung Verwendung finden sollen, werden ausschließlich auf Basis von Alkyl- und/oder Alkenylpolyglycolethem erhalten, die zunächst einen Block von Ethylenoxid-Einheiten und dann einen geringen Anteil von Propylenoxid-Einheiten aufweisen. Besonders bevorzugt sind hierbei Mischether auf Basis von Alkyl- und/oder Alkenylpolyglycolethem mit durchschnittlich 4 bis 12 Mol Ethylenoxid und 0,5 bis 1,5 Mol Propylenoxid.

### Alkyl-Endgruppenverschluß

Zum Alkyl-Endgruppenverschluß wird der Alkohol mit einem Überschuß Dialkylcarbonat, vorzugsweise Dimethylcarbonat in 10 min bis 1 d , vorzugsweise 30 min bis 5 h und insbesondere 1 bis 3 h bei 90 bis 150 °C, verestert. Nach Ablauf der Reaktion wird das überschüssige Dialkylcarbonat abdestilliert und im Anschluß bei 150 bis 300 °C, vorzugsweise 180 bis 220 °C, in 10 min bis 10 h, vorzugsweise 1 bis 3 h der Ether hergestellt. Alternativ kann die Herstellung des Ethers in einem Schritt (ohne vorherige Veresterung) in einem Autoklav bei 150 bis 300 °C, vorzugsweise 180 bis 220 °C, unter Eigendruck erfolgen. Das Entfernen des Dialkylcarbonat / Methanol-Gemisches erfolgt im Anschluß. Man erhält alkyl- und vorzugsweise methyl-endgruppenverschlossenen Alkyl- und/oder Alkenylether in einer Ausbeute von mindestens 90 %.
Es hat sich als vorteilhaft erwiesen, Alkohole und Alkylierungsmittel im molaren Verhältnis von 1 : 1 bis 1 : 100, vorzugsweise 1 : 3 bis 1 : 50 und insbesondere 1 : 9 bis 1 : 20 einzusetzen.
Als Katalysator für beide Varianten haben sich Metalloxide als vorteilhaft erwiesen. Diese werden in Mengen von 0.5 bis 100 und vorzugsweise 1 bis 10 Gew.-% eingesetzt (vgl. **EP 0476784 A1**).

### Gewerbliche Anwendbarkeit

Die Alkylmischether insbesondere die Methylmishether sind besonders schaumarm und zeichnen sich durch sehr gute entschäumende Eigenschaften, hohe Reinigungsleistung sowie gute ökotoxikologische Verträglichkeit aus. Demnach bestand ein weiteren Gegenstand der Erfindung in der Verwendung von alkyl- und vorzugsweise methyl-endgruppenverschlossenen Alkyl- und/oder Alkenylpolyglycolethern nach Anspruch 1 zur Herstellung von schaumarmen Reinigungsmitteln sowie Entschäumern für die Zucker- und Hefeindustrie, in denen sie in Mengen von 1 bis 50, vorzugsweise 5 bis 25 Gew.-% - bezogen auf die Mittel - enthalten sein können, wie beispielsweise:
- Mittel für die maschinelle Flaschenreinigung, insbesondere für die Reinigung von Bier- und Milchflaschen,
- Mittel für die Reinigung harter Oberflächen, insbesondere für das 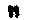 Cleaning-in-place" (CIP) sowie
- Mittel für die Entschäumung von Zwischenprodukten bei der Zucker- und Hefeherstellung.

Unter Reinigungsmitteln werden im Zusammenhang mit der Erfindung einmal die zur direkten Anwendung auf die zu reinigenden Substrate bestimmten wäßrigen Lösungen verstanden, daneben umfaßt der Begriff aber auch die zur Herstellung der Anwendungslösungen bestimmten Konzentrate und gegebenenfalls festen Mischungen. Die gebrauchsfertigen Lösungen können sauer bis stark alkalisch eingestellt sein und werden in der Regel bei Temperaturen von etwa 20 bis 90 °C eingesetzt.

### Hilfs- und Zusatzstoffe

Die Mittel können weitere Hilfs- und Zusatzstoffe und insbesondere weitem anionische, nichtionische, kationische und/oder amphotere bzw. zwitterionische Tenside enthalten.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

An Buildern und Komplexbildnern können die Mittel vor allem Alkalimetallorthophosphate, -polymerphosphate, -silica- te, -borate, -carbonate, -polyacrylate und -gluconate, sowie Zeolithe, Schichtsilicate, Citronensäure, Nitrilotriessigsäure, Ethylendiamintetraessigsäure, 1-Hydroxyalkan-1,1-diphosphonsäuren, Ethylendiamintetra-(methylenphosphonsäure) sowie Phosphonoalkanpolycarbonsäuren wie z.B. Phosphonobutantricarbonsäure bzw. Alkalimetallsalze dieser Säuren enthalten.

Hochalkalische Reinigungsmittel, insbesondere für die Flaschenreinigung, können ferner beträchtliche Mengen Ätzalkali in Form von Natrium - und/oder Kaliumhydroxid aufweisen. Wenn besondere Reinigungseffekte gewünscht werden, können die Mittel zudem organische Lösungsmittel, beispielsweise Alkohole, Benzinfraktionen sowie freie Alkanolamine enthalten.

Die folgenden Beispiele sollen den Gegenstand der Erfindung näher erläutern, ohne ihn darauf einzuschränken.

### Beispiele

### 1. Herstellvorschriften für Mischether

a) Alkohol A : in der Formel **(I)** steht R¹ für einen Alkylrest mit 8 Kohlenstoffatomen, n für 4 und m für 0.
b) Alkohol B : in der Formel **(I)** steht R¹ für ein Alkylrest mit 12 bis 14 Kohlenstoffatomen, n für 5 und m für 3.

- Al₂0₃⁶⁰basisch (Fa. Merck)
- Hydrotalcit KW 2000 (Fa. Kyowa)

### 1. Methylierung des Alkohols A mit Dimethylcarbonat (DMC), Katalysator: Al₂O₃

In einen 250 ml Autoklav werden 6 g des Alkohols A in 11,2 ml DMC gelöst und 6 g Aluminiumoxid hinzu gegeben (1:2:1 Gew.-%). Die Mischung wird unter Stickstoffstrom erwärmt und bei 200 °C 1 Stunde gerührt (Druckaufbau: 38 bar). Der Autoklav wird abgekühlt und anschließend geöffnet. Dieses Verfahren wird zweimal wiederholt, indem jedesmal 11,2 ml DMC zugegeben werden (Umsetzung > 95%). Danach wird der Katalysator abfiltriert, das Reaktionsgemisch mit Methanol abgewaschen und das überschüssige DMC und das Methanol unter Vakuum abdestilliert. Man erhält 5,8 g Methyletherderivat (viskoses, leicht gelbes Öl). Ausbeute > 95%.

### 2. Zwei-Stufe Methylierung des Alkohols A mit DMC, Katalysator: Hydrotalcit

In einem 250 ml Autoklav werden 1 g des Alkohols A in 15 ml DMC gelöst und 0,1 g Hydrotalcit KW-2000 hinzu gegeben (1:16:0,1 Gew.-%). Die Mischung wird unter Stickstoffstrom erwärmt und bei 120 °C 1 Stunde gerührt (Druckaufbau: 3 bar). Der Autoklav wird abgekühlt und anschließend geöffnet. Der Inhalt (>96% Methylcarbonatderivat) wird in eine Destillationsapparatur transferiert und erwärmt (Sumpftemperatur = 120 °C). Man erhält 13 ml Destillat, welches anschließend auf 200 °C erwärmt wird. Nach 60 Minuten wird der Rückstand filtriert, mit Methanol abgewaschen und getrocknet. Der Methylether (gelbes Öl) wird in >90%iger Ausbeute erhalten.

### 3. Methylierung des Alkohols B mit DMC, Katalysator: Hydrotalcit

In einem 250 ml Autoklav werden 1 g des Alkohols B in 10 ml DMC gelöst und 1 g Hydrotalcit KW-2000 hinzu gegeben (1:10,7:1 Gew.-%). Die Mischung wird unter Stickstoffstrom erwärmt und bei 200 °C 4 Stunden gerührt (Druckaufbau: 28 bar). Der Autoklav wird abgekühlt und anschließend geöffnet. Danach wird der Katalysator abfiltriert, das Reaktionsgemisch mit Methanol abgewaschen und das überschüssige DMC und das Methanol unter Vakuum abdestilliert. Das Methyletherderivat wird als viskoses gelbes Öl erhalten (Ausbeute > 95%).

### 4. Methylierung von 1-Octanol mit DMC, Katalysator: Al₂O₃

In einem 250 ml Autoklav werden 12 g 1-Octanol in 11,7 ml DMC gelöst und 12 g Aluminiumoxid hinzu gegeben (1:2:1 Gew.-%). Die Mischung wird unter Stickstoffstrom erwärmt und bei 200 °C 1 Stunde gerührt (Druckaufbau: 37 bar). Der Autoklav wird abgekühlt und anschließend geöffnet. Dieses Verfahren wird fünfmal unter Zugabe von 11,7 ml DMC wiederholt. Danach wird der Katalysator abfiltriert, das Reaktionsgemisch mit Methanol abgewaschen und das überschüssige DMC und das Methanol unter Vakuum abdestilliert. Man erhält 13.2 g Methyloctylether (Ausbeute >99%).

### II. Bestimmung der Entschäumerwirkung

**Methode**. In einem doppelwandigen 2-l-Meßzylinder werden 300 ml einer 1 Gew.-%igen Natriumhydroxidlösung auf 20 bzw. 65 °C temperiert. Anschließend wird der jeweils ausgewählte schaumdrückende Zusatzstoff in den nachfolgend angegebenen Mengen zugesetzt. Mit Hilfe einer Laborschlauchpumpe wird die Flüssigkeit mit einer Umwälzgeschwindigkeit von 4 l/min umgepumpt. Dabei wird die Prüflösung ca. 5 mm über dem Boden des Meßzylinders mit Hilfe eines Glasrohres, das mit einer Pumpe verbunden ist, angesaugt und über ein zweites Glasrohr, das an der 2000-ml-Marke angebracht ist, in freiem Fall zurückgeführt.
Nach 30 s dosiert man zunächst 1 ml einer 1 Gew.-%igen wäßrigen Lösung von Tetrapropylenbenzolsulfonat-Triethanolammonium-Salz (nachfolgend als 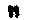 Testschäumer TS" bezeichnet) in die Flotte und bestimmt nach weiteren 30 s das entstandene Volumen, das durch Flüssigkeit und Schaum gebildet wird. In Zeitabständen von jeweils 1 min wird nachfolgend weiterer Testschäumer zudosiert und das nach 30 s entstandene Schaum/ Flüssigkeits-Volumen bestimmt. Diesen stufenförmigen Zyklus von Zudosierung des Testschäumers und Schaummessung setzt man solange fort, bis die Tensidlösung im Meßzylinder auf 2000 ml aufgeschäumt ist.
Die Ergebnisse sind in den Tabellen 2 zusammengefaßt.

**Tabelle 1**

| **Methyl und Butylmischether** | | | | | |
|---|---|---|---|---|---|
| Bsp. | R¹ | Mol EO | Mol PO | Mol EO | EGV |
| M1 | Octyl | 4 | 1 | - | Methyl |
| M2 | Dodecyl | 7 | 1,2 | - | Methyl |
| M3 | 2-Hexyldodecyl | 10 | 1,2 | 1,2 | Methyl |
| M4 | Octyl | - | 1 | 4 | Methyl |
| M5 | Octyl | 2 | 1 | 2 | Methyl |
| M6 | Dedecyl | - | 1,2 | 7 | Methyl |
| M7 | Dedecyl | 7 | - | - | Butyl |
| M8 | Dedecyl | 3 | 1,2 | 4 | Butyl |

**Tabelle 2**

| **Entschäumerwirkung der Methylmischether (20 °C), Mengenangaben in ml** | | | | | | | | |
|---|---|---|---|---|---|---|---|---|
| **TS** | **M 1** | **M 2** | **M 3** | **M 4** | **M 5** | **M 6** | **M 7** | **M 8** |
| 0 | 300 | 300 | 300 | 300 | 300 | 300 | 300 | 300 |
| 1 | 300 | 300 | 300 | 300 | 300 | 300 | 300 | 300 |
| 2 | 300 | 300 | 300 | 300 | 300 | 300 | 300 | 300 |
| 3 | 300 | 300 | 300 | 320 | 320 | 300 | 300 | 300 |
| 4 | 340 | 320 | 300 | 340 | 330 | 320 | 320 | 320 |
| 5 | 380 | 360 | 300 | 390 | 380 | 360 | 360 | 360 |
| 6 | 400 | 390 | 300 | 440 | 440 | 390 | 390 | 390 |
| 7 | 420 | 410 | 420 | 470 | 480 | 430 | 430 | 430 |
| 8 | 420 | 410 | 420 | 490 | 490 | 450 | 450 | 450 |
| 9 | 460 | 430 | 430 | 530 | 520 | 470 | 470 | 470 |
| 10 | 480 | 460 | 470 | 570 | 550 | 490 | 480 | 490 |
| 11 | 500 | 490 | 490 | 590 | 590 | 520 | 520 | 520 |
| 12 | 540 | 520 | 510 | 630 | 630 | 560 | 550 | 560 |
| 13 | 580 | 550 | 550 | 690 | 680 | 590 | 590 | 590 |
| 14 | 600 | 580 | 570 | 780 | 750 | 640 | 630 | 620 |
| 15 | 660 | 650 | 660 | 950 | 940 | 700 | 720 | 700 |
| 16 | 720 | 700 | 710 | 1150 | 1100 | 890 | 820 | 880 |
| 17 | 800 | 790 | 810 | 1700 | 1650 | 990 | 990 | 980 |
| 18 | 1000 | 950 | 990 | 2000 | 2000 | 1300 | 1290 | 1400 |
| 19 | 1300 | 1250 | 1270 | | | 2000 | 1650 | 2000 |
| 20 | 1680 | 1500 | 1530 | | | | 2000 | |
| 21 | 2000 | 2000 | 2000 | | | | | |

**Tabelle 3**

| **Entschäumerwirkung der Methylmischether (65 °C), Mengenangaben in ml** | | | | | | | | |
|---|---|---|---|---|---|---|---|---|
| | **M1** | **M2** | **M3** | **M4** | **M5** | **M6** | **M7** | **M8** |
| Testschäumer bis 2000 ml Volumen | 20 | 20 | 20 | 15 | 14 | 15 | 18 | 15 |

## Patentansprüche

1. Verfahren zur Herstellung von alkyl-endgruppenverschlossenen Alkyl- und/oder Alkenylethern, **dadurch gekennzeichnet**, daß man Alkohole der Formel **(I)**, 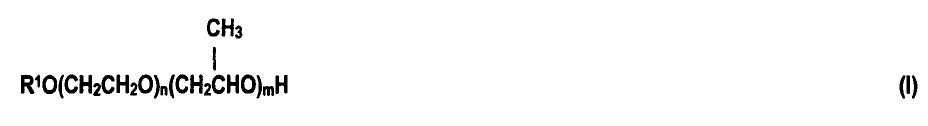 in der R¹ für einen linearen oder verzweigten, gesättigten oder ungesättigten Alkyl-, Alkenyl- und/oder Acylrest mit 6 bis 22 Kohlenstoffatomen und/oder gegebenenfalls mit einer Gruppe R² substituierten Kohlenwasserstoffrest, in der R² für H, C₁-₁₂ Alkyl, Glyceryl oder den Rest eines Alkylglycols mit 2 bis 6 Kohlenstoffatomen, n für Zahlen von 0 bis 15 und m für Zahlen von 0 bis 10 steht, mit Dialkylcarbonaten der Formel **(II)**, 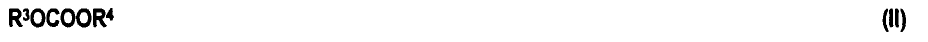 in der R³ und R⁴ unabhängig voneinander für einen Alkylrest mit 1 bis 8 Kohlenstoffatomen steht, umsetzt.

2. Verfahren nach Anspruch 1 **dadurch gekennzeichnet**, daß man Alkoholethoxylate der Formel **(I)** einsetzt, in der R¹ für einen linearen oder verzweigten, gesättigten oder ungesättigten Alkyl- und/oder Alkenylrest mit 6 bis 22 Kohlenstoffatomen steht.

3. Verfahren nach den Ansprüchen 1 und/oder 2, **dadurch gekennzeichnet**, daß man Alkohotethoxylate der Formel **(I)** einsetzt, in der R¹ für einen Alkyl- und/oder Alkenylrest mit 8 bis 18 Kohlenstoffatomen steht.

4. Verfahren nach mindestens einem der Ansprüche 1 bis 3, **dadurch gekennzeichnet**, daß man Guerbetalkoholethoxylate der Formel **(I)** einsetzt, in der R¹ für einen in 2-Stellung verzweigten Alkylrest mit 16 bis 20 Kohlenstoffatomen steht.

5. Verfahren nach mindestens einem der Ansprüche 1 bis 4, **dadurch gekennzeichnet**, daß man Alkoholethoxylate der Formel **(I)** mit Dimethylcarbonat umsetzt.

6. Verwendung von alkyl-endgruppenverschlossenen Alkyl- und/oder Alkenylpolyglycolethern nach den Ansprüchen 1 bis 5 zur Herstellung von schaumarmen Reinigungsmitteln sowie Entschäumern für die Zucker- und Hefeindustrie.

7. Verwendung von methyl-endgruppenverschlossenen Alkyl- und/oder Alkenylpolyglycolethern nach den Ansprüchen 1 bis 6 zur Herstellung von schaumarmen Reinigungsmitteln sowie Entschäumern für die Zucker- und Hefeindustrie.
